# EUROPEAN PATENT APPLICATION

(11) **EP 4 353 077 A1**
(43) Date of publication of application: **17.04.2024**
(21) Application number: 22201496.1
(22) Date of filing: 14.10.2022
(51) Int. Cl.: A01H 1/00, C12N 9/22, C12N 15/82

(54) **OSBCAT2 GENE CONTROLLING DROUGHT TOLERANCE OF PLANT AND USES THEREOF**

(71) Applicant: Lasemilla Co., Ltd., Pyeongchang 25354 (KR)
(72) Inventor: KIM, Ju-Kon, 25315 Pyeongchang (KR); SEO, Jun Sung, 25354 Pyeongchang (KR); OH, Se-Jun, 04166 Seoul (KR); YOON, Hobin, 11129 Pocheon (KR)
(74) Representative: Cabinet Nony

(57) **Abstract**

The present invention relates to *OsBCAT2* (*BRANCHED-CHAIN AMINO ACID AMINOTRANSFERASE* 2) gene controlling drought stress of plant and uses thereof.

## Description

### TECHNICAL FIELD

The present invention relates to *OsBCAT2* (*Oryza sativa BRANCHED-CHAIN AMINO ACID AMINOTRANSFERASE 2)* gene controlling drought stress of plant and uses thereof.

This work was supported by the Starting growth Technological R&D Program (TIPS Program (No. S3307687)) funded by the Ministry of SMEs and Startups (MSS, Korea) in 2022.

### BACKGROUND ART

Unpredictable weather events caused by climate change occur worldwide. Abiotic stress is a natural incident with disastrous consequences for nearby living things. Moreover, extreme weather disasters lead to crop yield decreases. The drought stress significantly influenced international crop production. According to the reports, in rice *(Oryza sativa.* L.) plants, drought stress inflicts irreversible damage during the whole period of growth and decreases the grain yield drastically. Specifically, drought stress in the vegetative stage of rice (Yangliangyou 6, Hanyou 113) caused a yield reduction of 23-24%.

Plants have various strategies to combat abiotic stress. At the molecular level, plants regulate the expression of genes involving in stress responses through the stress-specific signaling pathway. Physiological responses lead to morphological and endogenous biochemical adjustments. For instance, plant amino acids play an essential role in minimizing the damage by abiotic stress. So far, proline is best characterized amino acid that accumulates in plant cells to confront a wide range of abiotic stress. It has been documented that proline accumulates in response to drought, salt, heat, low temperature, and nutrient deficient stress in various plants. γ-Aminobutyric acid (GABA) alleviates drought and heat stress through regulating antioxidant genes in sunflower. Besides, glycine, taurine, arginine, and serine were reported as stress-induced amino acids which act as osmolytes. Interestingly, among plant amino acids, Branched-chain amino acids (BCAA), leucine (Leu), isoleucine (Ile), and valine (Val) have been reported to accumulate under stress conditions in plants. Fold change value of BCAA quantity under drought conditions was higher than that of proline, a well-known drought inducible amino acid.

BCAA has a critical role in protein biosynthesis and plant development. Moreover, they are also involved in the formation of diverse secondary metabolites, such as cyanogenic glycosides, glucosinolate, and acyl-sugars. Branched-chain amino acids synthesis is carried out with two distinct substrates. The preliminary pathway of valine and leucine starts with pyruvate created by glycolysis. On the other hand, the synthesis of isoleucine starts with 2-ketobutyrate after the threonine deamination by threonine deaminase (TD, EC 4.2.1.16). The first engaged enzyme for Val, Leu production, is acetohydroxyacid synthase (AHAS also known as acetolactate synthase ALS, EC 2.2.1.6), which catalyzes both two pyruvate molecules for the constitution of acetolactate for Val and α-ketobutyrate with one pyruvate to form acetohydroxybutyrate as a precursor of isoleucine. Ketol-acid reductoisomerase (KARI, EC 1.1.1.86), and dihydroxy-acid dehydratase (DHAD, EC 4.2.1.9), consecutively catalyze a fundamental two-step reaction for BCAA synthesis. The branched-chain amino acid aminotransferase (BCATs, EC 2.6.1.42) is in charge of the final step of BCAA biosynthesis in plastids.

BCAA catabolic reactions mainly occur in mitochondria. BCAT is an enzyme involved in the first step of degradation and the last step in synthesizing BCAA. BCAT, which belongs to Class IV of the pyridoxal 5'phosphate-dependent aminotransferases, converts Val, Ile, or Leu to α-keto acids KIV, KIC, or KMV. Branched-chain keto acid dehydrogenase (BCKDH also known as BCKDH complex, EC 1.2.4.4) catalyzes the branched-chain α-keto acids to isobutyryl-CoA, isovaleryl-CoA, or methylbutyryl-CoA (Taylor et al. 2004). Then, Isovaleryl-CoA dehydrogenase (IVD, EC 1.3.99.10), identified in several plant species, catalyzes acyl-CoA to enoyl-CoA. Although this enzyme has not been fully elucidated, the substrate specificity appears to be different among these proteins of other species.

Among these catabolic enzymes, Interestingly, plant species have various BCAT isoform families. In *Arabidopsis thaliana* and *Solanum lycopersicum,* seven and six distinct isoforms of BCAT have been validated, respectively. It has been reported that AtBCAT2 (At1g10070), S1BCAT1 (SGN-U569828), and S1BCAT2 (SGN-U569830) are localized to mitochondria, where it conducts a vital function in BCAA catabolism. Nevertheless, the pathways related to gene and catabolic protein networks have not been fully elucidated in plants.

Meanwhile, in Korean Patent Publication No. 2021-0063574, "Drought resistant transgenic plant inhibiting AtPR5K2 protein expression" is disclosed, and in Korean Patent Publication No. 2022-0083464, "Novel branched-chain amino acid aminotransferase variant and method of producing L-isoleucine using the same" is disclosed. However, *OsBCAT2* gene controlling plant drought tolerance of the present invention and uses thereof have not been described.

### DETAILED DESCRIPTION OF THE INVENTION

### TECHNICAL PROBLEMS TO BE SOLVED

The present invention is devised according to the necessities that are explained in the above. Specifically, based on previous research examining the role played by branched-chain amino acids (BCAA) on plant tolerance under abiotic stress conditions, inventors of the present invention studied the function of *OsBCAT2* (*BRANCHED-CHAIN AMINO ACID AMINOTRANSFERASE 2)* gene, which is derived from rice.

By using CRISPR-Cas9 system, the inventors of the present invention prepared loss of function rice *OsBCAT2* mutants (i.e., *osbcat2*). In addition, as a result of examining the drought tolerance exhibited by the *osbact2,* the inventors found that *osbact2* mutants show enhanced tolerance to drought and enhanced grain yield over those of wild type under various drought stress conditions. The present invention is completed accordingly.

### TECHNICAL MEANS FOR SOLVING THE PROBLEMS

To solve the problems that are described in the above, the present invention provides a method of controlling tolerance to drought stress in a plant comprising regulating expression of a gene encoding OsBCAT2 (BRANCHED-CHAIN AMINO ACID AMINOTRANSFERASE 2) protein derived from rice in a plant cell.

The present invention further provides a method of producing a transgenic plant having controlled tolerance to drought stress comprising: transforming a plant cell with a recombinant vector containing a gene encoding OsBCAT2 protein derived from rice; and regenerating a transgenic plant from the transformed plant cell.

The present invention further provides a transgenic plant having controlled tolerance to drought stress produced by the aforementioned method of producing a transgenic plant, and a transgenic seed thereof.

The present invention further provides a method of producing a genome-edited rice plant having enhanced tolerance to drought stress comprising: (a) carrying out genome editing by introducing guide RNA specific to target nucleotide sequence in a gene encoding OsBCAT2 protein derived from rice, and endonuclease protein to a rice plant cell; and (b) regenerating a plant from the genome-edited rice plant cell.

The present invention further provides a genome-edited rice plant having enhanced tolerance to drought stress produced by the aforementioned method of producing a genome-edited rice plant, and a genome-edited seed thereof.

The present invention still further provides a composition for controlling tolerance to drought stress in a plant comprising, as an effective component, a gene encoding OsBCAT2 protein derived from rice.

### ADVANTAGEOUS EFFECT OF THE INVENTION

Because having crops with tolerance to environmental stress is considered to be very important in the field of agriculture, it is expected that *OsBCAT2* (branched-chain amino acid aminotransferase 2) gene derived from rice, which is described in the present invention, can be advantageously used for enhancing the plant tolerance to drought stress.

### BRIEF DESCRIPTION OF THE DRAWINGS

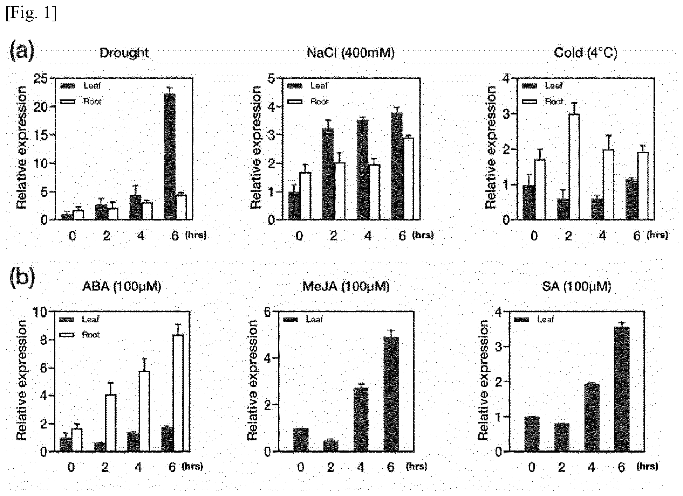

Figure 1 shows expression patterns of *OsBCAT2* in response to various abiotic stress and hormones. (a) The relative expression level of *OsBCAT2* under drought (air-drying), Salt (400 mM NaCl), low temperature (4°C). (b) The relative expression level of *OsBCAT2* after treatment with 100 µM of abscisic acid, methyl jasmonate, and salicylic acid, respectively. The rice uibiquitin1 (Os06g068100) was used as an internal standard for normalization. The error bar indicates the standard deviation of three replicate measurements.

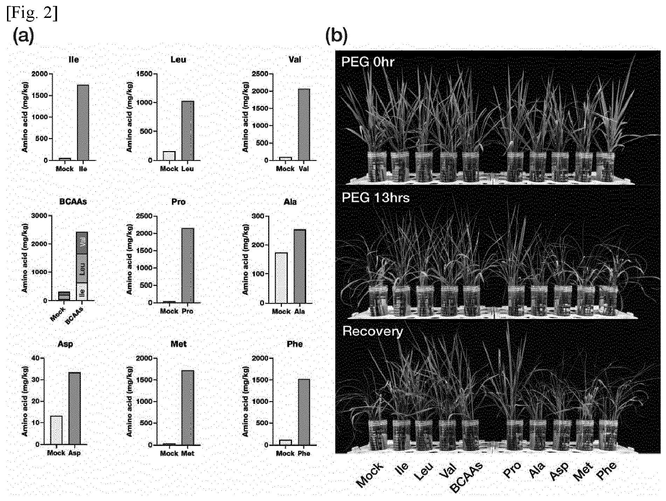

Figure 2 shows osmotic stress phenotype induced by PEG after FAA (free amino acid) treatment.

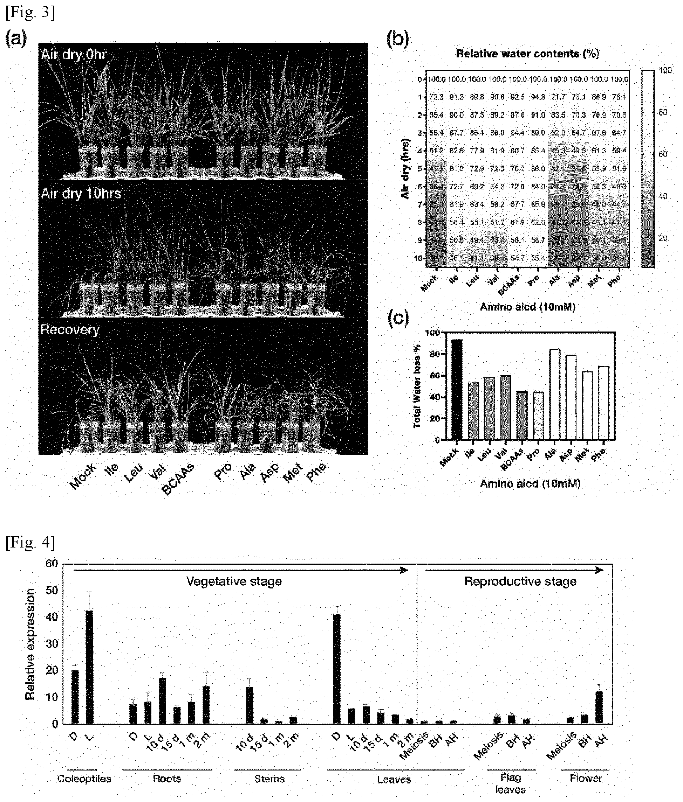

Figure 3 shows the phenotype of FAA-treated NT plants induced by air drying. (a) Air-drying induced abiotic stress phenotype (n=10). (b) Relative water content change by time-course during air-dry treatment after amino acid treatment in the NT plant. (c) Total water loss during air-drying.
Figure 4 shows the expression pattern of *OsBCAT2* genes at various developmental stages and tissue. Total RNA was extracted at various tissue and developmental stages (d, day; w, week; m, month; M, meiosis; BH, before heading; AH, after heading). The expression level was analyzed by quantitative RT-PCR analysis of *OsBCAT2.* The rice *UIBIQUITIN1* (Os06g068100) was used as an internal standard for normalization. The error bar indicates the standard deviation of three replicate measurements.

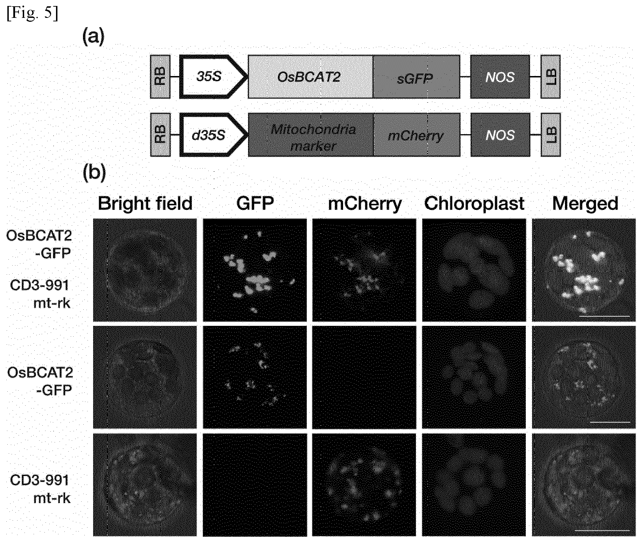

Figure 5 shows subcellular localization of the OsBCAT2 protein in rice protoplasts. *35S::OsBCAT2-GFP* and CD3-991-mCherry as mitochondria markers were co-transfected and transiently expressed in rice leaf protoplasts. Scale bar, 10 µm

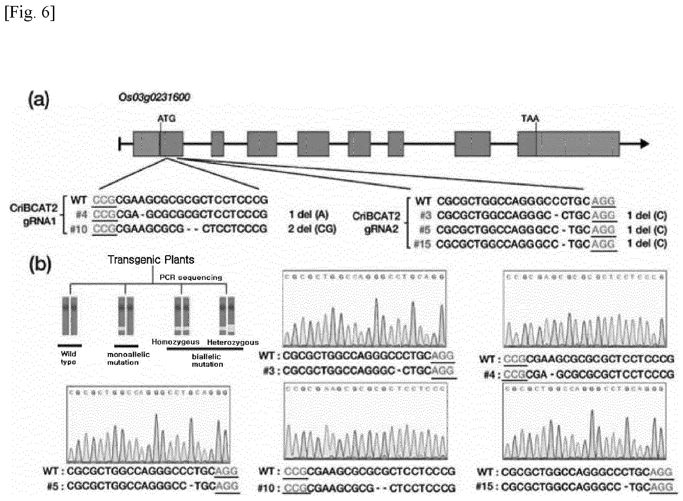

Figure 6 shows construction for loss of function *OsBCAT2* by CRISPR-Cas9 systems and their genotype. (a) Two single guide RNA were designed in the first exon region to derive the premature stop codon. (b) Mutation pattern on *osbcat2* mutant #3, #4, #5, #10 and #15. Underlined marking stands for protospacer adjacent motif (PAM), and base deletion is marked with a hyphen.

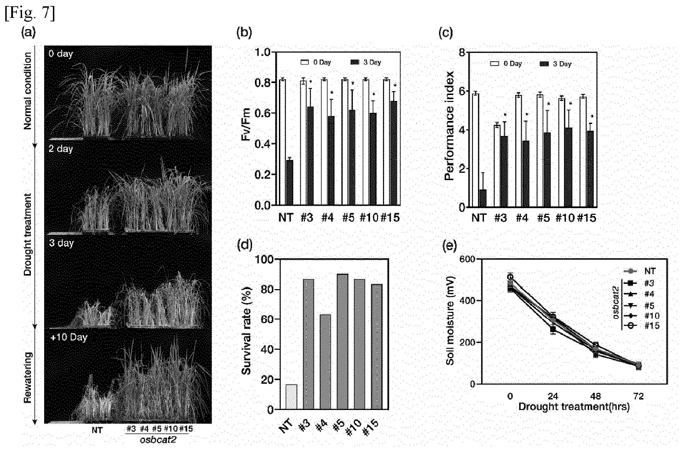

Figure 7 shows loss of function *OsBCAT2* confers drought tolerance. (a) Five different *osbcat2* lines were used. Visual results from sequential drought stress and re-watered treatments in the greenhouse under normal conditions for a month, including non-transgenic (NT) plants. The number of days on the images indicates the duration of the drought and re-watering, (b) Photochemical efficiency measured during the drought treatment period, Fv/Fm values indicates the quantum yield of photosystem II. (c) Performance index shows the efficiency of the two photosystems (I and II) carrying out potential photosynthesis of plant leaves(n=10). (d) Survival rates were counted after recovery via re-watering after drought treatment. (e) Soil moisture during the time-course drought treatment period (n=20). Asterisks denote statistically significant differences compared to NT unpaired student's t-tests as follows: ^{∗}P < 0.05

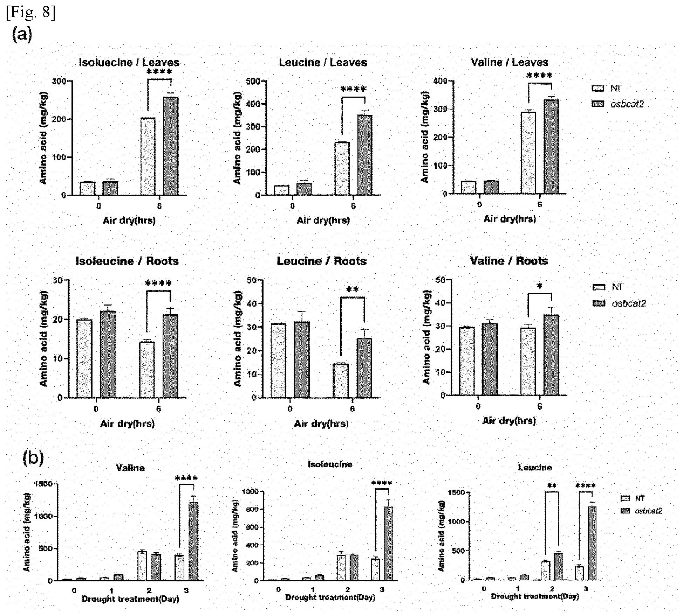

Figure 8 shows loss of *OsBCAT2* accumulated BCAA content under various drought conditions in shoot and roots. (a) BCAA content in *osbcat2* and NT before and after air dry treatment. (b) BCAA content accumulation by time-course drought stress in *osbcat2* and NT. The error bar indicates the standard deviation, the technical repeat of each experiment was repeated three times. Asterisks denote statistically significant differences compared to NT unpaired student's t-tests as follows: ^{∗}P < 0.05, ^{∗∗}P < 0.01, ^{∗∗∗∗}P < 0.0001.

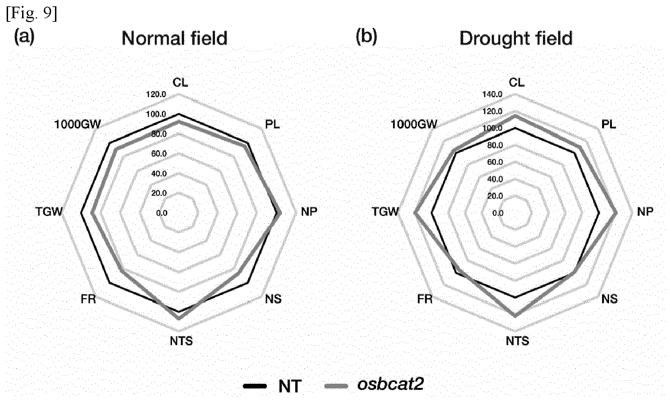

Figure 9 shows the yield of *osbcat2* improved under drought conditions. The agronomic trait of *osbcat2* and NT in (a) normal and (b) drought field conditions. Mean values from NT plants in Table 3 were allocated a reference value of 100 %. CL, culm length; PL, panicle length; NP, number of panicles; NS, number of spikelets; NTS, number of total spikelets; FR, filling rate; TGW, total grain weight; 1000GW, 1000-grain weight.

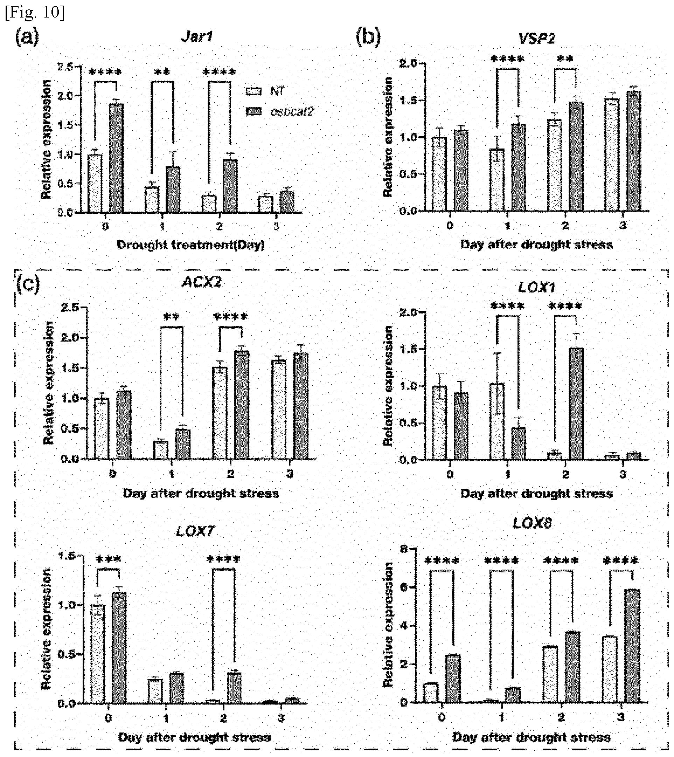

Figure 10 shows transcription analysis of JA-related gene in drought time course treatment in greenhouse condition of *osbcat2* and NT plants. Relative expression of (a) a critical enzyme for JA-Ile formation, (b) JA-response gene and (c) JA-signaling core genes. The rice uibiquitin1 (Os06g068100) was used as an internal standard for normalization. The error bar indicates the standard deviation of three replicate measurements. Asterisks denote statistically significant differences compared to NT unpaired student's t-tests as follows: ^{∗}P<0.05, ^{∗∗}P < 0.01, ^{∗∗∗∗}P < 0.0001

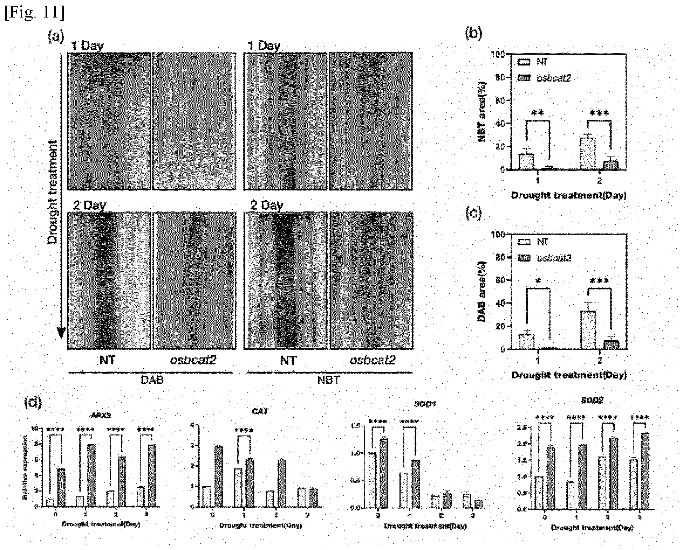

Figure 11 shows loss of function *OsBCAT2* enhances the reactive oxygen species (ROS)-scavenging capacity under time-course water deficit conditions. (a) The histochemical staining assay through 3,3-diaminobenzidine (DAB) and nitro blue tetrazolium (NBT) for the detection of H₂O₂ and O₂⁻ in osbcat2 compared to NT under drought conditions, and (b), (c) its stained area. (d) Relative expression of antioxidant genes, *L-ASCORBATE PEROXIDASE2* (*APX2*), *CATALASE* (*CAT*), and *SUPEROXIDE DISMUTASE1,2* (*SOD1,2*). The rice *UIBIQUITIN 1* (Os06g068100) was used as an internal standard for normalization. The error bar indicates the standard deviation of three replicate measurements. Asterisks denote statistically significant differences compared to NT unpaired student's t-tests as follows: ^{∗}P<0.05, ^{∗∗}P < 0.01, ^{∗∗∗∗}P < 0.0001

### BEST MODE(S) FOR CARRYING OUT THE INVENTION

To achieve the object of the present invention, the present invention provides a method of controlling tolerance to drought stress in a plant comprising regulating expression of a gene encoding *OsBCAT2* (branched-chain amino acid aminotransferase 2) protein derived from rice in a plant cell.

With regard to the method of controlling tolerance to drought stress according to one embodiment of the present invention, the OsBCAT2 protein derived from rice may preferably consist of the amino acid sequence of SEQ ID NO: 2, but it is not limited thereto.

Also included in the scope of the OsBCAT2 protein derived from rice according to the present invention are the protein having the amino acid sequence represented by SEQ ID NO: 2, and functional equivalents thereof. As described herein, the term "functional equivalents" means a protein which has, as a result of addition, substitution, or deletion of an amino acid, at least 70%, preferably at least 80%, more preferably at least 90%, and even more preferably at least 95% sequence homology with the amino acid sequence represented by SEQ ID NO: 2, and it indicates a protein which exhibits substantially the same physiological activity as the protein represented by SEQ ID NO: 2. The expression "substantially the same physiological activity" indicates an activity of controlling plant tolerance to drought stress.

Also included in the present invention is a gene which encodes OsBCAT2 protein derived from rice, and the gene has the scope to encompass all genomic DNA, cDNA, and synthetic DNA which encode OsBCAT2 protein derived from rice. Preferably, the gene encoding OsBCAT2 protein derived from rice described in the present invention may comprise the *OsBCAT2* nucleotide sequence derived from rice that is represented by SEQ ID NO: 1. Furthermore, homologs of the aforementioned sequence are also within the scope of the present invention. Specifically, the aforementioned gene may include a nucleotide sequence which has 70% or higher, more preferably 80% or higher, even more preferably 90% or higher, and most preferably 95% or higher sequence homology with the nucleotide sequence of SEQ ID NO: 1. The "sequence homology %" of the polynucleotide is identified by comparing two sequences that are optimally aligned. In this regard, a part of the polynucleotide in comparative region may comprise an addition or a deletion (i.e., a gap) compared to a reference sequence (without any addition or deletion) in the optimized alignment of the two sequences.

The method of controlling plant tolerance to drought stress according to the present invention may involve the inhibition of expression of a gene encoding OsBCAT2 protein derived from rice in plant cell so as to have higher plant tolerance to drought stress compared to a non-transgenic plant, but it is not limited thereto.

According to one embodiment of the present invention, the inhibition of expression of a gene encoding OsBCAT2 protein derived from rice can be an inhibition (suppression) of the gene encoding OsBCAT2 protein derived from rice based on insertion of T-DNA to the gene encoding OsBCAT2 protein derived from rice, induction of mutation using endogenous transposon or irradiation of X-ray or γ-ray, or use of RNAi or antisense RNA, or CRISPR/Cas9 gene editing system, but it is not limited thereto. Any method typically employed in the pertinent art to have inhibited gene expression can be possibly used.

With regard to the recombinant vector of the present invention, the promoter is a promoter which is suitable for transformation. Preferably, it may be any one of CaMV 35S promoter, actin promoter, ubiquitin promoter, pEMU promoter, MAS promoter or histone promoter. Preferably, it may be CaMV 35S promoter, but it is not limited thereto.

The recombinant expression vector may preferably contain one or more selective marker. The selective marker is a nucleotide sequence having a property of allowing vector selection by a common chemical method. Any gene that can be used for identifying transformed cells from non-transformed cells can be a selective marker. The marker gene can be a dominant drug-resistance gene, but it is not limited thereto.

In case of transforming an eukaryotic cell with the vector of the present invention, yeast (*Saccharomyce cerevisiae*), an insect cell, human cell (e.g., CHO (Chinese hamster ovary) cell line, W138, BHK, COS-7, 293, HepG2, 3T3, RIN and MDCK cell line), a plant cell, or the like can be used as a host cell. Preferably, the host cell is a plant cell.

The present invention further provides a method of producing a transgenic plant having controlled tolerance to drought stress comprising: transforming a plant cell with a recombinant vector containing a gene encoding OsBCAT2 protein derived from rice; and regenerating a transgenic plant from the transformed plant cell.

With regard to the method of producing a transgenic plant according to the present invention, the OsBCAT2 protein derived from rice may preferably include a protein consisting of the amino acid sequence of SEQ ID NO: 2, and functional equivalents thereof. In this regard, the detailed information is the same as those described above.

Further, with regard to the method of producing a transgenic plant described in the present invention, the method for transforming a plant cell is the same as described in the above. As for the method for regenerating a transgenic plant from a transformed plant cell, a method well known in the pertinent art can be used. The transformed plant cell needs to be regenerated into a mature plant. Techniques for regeneration into a mature plant by culture of callus or protoplast are well known in the pertinent art for various species.

With regard to the method of producing a transgenic plant according to one embodiment of the present invention, by inhibiting the expression of the gene, which encodes OsBCAT2 protein derived from rice, in plant cell, higher plant tolerance to drought stress compared to a non-transgenic plant, i.e., wild type, can be obtained, but it is not limited thereto.

Inhibiting the expression of the gene which encodes OsBCAT2 protein derived from rice can be achieved by transforming a plant cell with a recombinant vector containing sense or antisense DNA, or microRNA for *OsBCAT2* gene derived from rice to inhibit the expression of the gene which encodes OsBCAT2 protein derived from rice. However, the inhibition is not limited thereto, and any gene expression inhibition technique well known in the pertinent art can be employed.

The present invention further provides a transgenic plant having controlled tolerance to drought stress, which is produced by the aforementioned method of producing a transgenic plant, and a transgenic seed thereof.

The transgenic plant according to the present invention is characterized in that, as expression of the gene encoding OsBCAT2 protein derived from rice is inhibited, the plant exhibits enhanced tolerance to drought stress. The plant can be a monocot plant such as rice, barley, wheat, rye, corn, sugar cane, oat, or onion, or a dicot plant such as *Arabidopsis thaliana,* potato, eggplant, tobacco, pepper, tomato, burdock, crown daisy, lettuce, balloon flower, spinach, chard, yam, carrot, water parsley, Chinese cabbage, cabbage, Raphanus sativus for. raphnistroides MAK, watermelon, oriental melon, cucumber, zucchini, gourd, strawberry, soybean, mung bean, kidney bean, or sweet pea. Preferably, it can be a monocot plant, and more preferably rice plant. However, it is not limited thereto.

The present invention further provides a method of producing a genome-edited rice plant having enhanced tolerance to drought stress comprising: (a) carrying out genome editing by introducing guide RNA specific to target nucleotide sequence in a gene encoding OsBCAT2 protein derived from rice, and enconuclease protein to a rice plant cell; and (b) regenerating a plant from the genome-edited rice plant cell.

As described herein, the term "genome/gene editing" means a technique for introducing a target-oriented mutation to a genome nucleotide sequence of a plant or an animal cell including human cell. Specifically, it indicates a technique for knock-out or knock-in of a specific gene by deletion, insertion, or substitution of one more nucleic acid molecules by DNA cutting, or a technique for introducing a mutation even to a non-coding DNA sequence which does not produce any protein. According to the purpose of the present invention, the genome editing may be an introduction of a mutation to a plant by using an endonuclease, for example, Cas9 (CRISPR associated protein 9) protein, and a guide RNA. Furthermore, the term "gene editing" may be interchangeably used with "gene engineering".

Furthermore, the term "target gene" described herein means part of DNA which is present in the genome of a plant and to be edited according to the present invention. Type of the target gene is not limited, and it may include a coding region and also a non-coding region. Depending on the purpose, a person who is skilled in the pertinent art may select the target gene according to the mutation of a plant that is desired to be produced by genome editing.

Furthermore, the term "guide RNA" described herein means a ribonucleic acid which includes RNA specific to a target DNA in nucleotide sequence encoding the target gene and, according to complementary binding between the whole or partial sequence of the guide RNA and the nucleotide sequence of target DNA, the guide RNA plays the role of guiding an endonuclease to the target DNA nucleotide sequence. The guide RNA represents two types of RNA, i.e., a dual RNA having crRNA (CRISPR RNA) and tracrRNA (trans-activating crRNA) as a constitutional element; or a single chain guide RNA (sgRNA) comprising the first site which includes a sequence that is fully or partially complementary to the nucleotide sequence in the target gene and the second site which includes a sequence interacting with RNA-guide nuclease, but those in the form in which the RNA-guide nuclease is active for the target nucleotide sequence are also within the scope of the invention without any limitation. Considering the type of an endonuclease to be used together, microorganisms from which the endonuclease is derived, or the like, the guide RNA may be suitably selected according to the technique that is well known in the pertinent art.

Furthermore, the guide RNA may be those transcribed from a plasmid template, those obtained by *in vitro* transcription (e.g., oligonucleotide double strand), or those obtained by synthesis, or the like, but it is not limited thereto.

Furthermore, with regard to the method of producing a genome-edited rice plant according to the present invention, the endonuclease protein may be one or more selected from a group consisting of Cas9, Cpf1 (CRISPR from Prevotella and Francisella 1), TALEN (Transcription activator-like effector nuclease), ZFN (Zinc Finger Nuclease), and a functional analog thereof. It may be preferably Cas9 protein, but it is not limited thereto.

Furthermore, the Cas9 protein may be one or more selected from a group consisting of Cas9 protein derived from *Streptococcus pyogenes,* Cas9 protein derived from *Campylobacter jejuni,* Cas9 protein derived from *S. thermophilus,* Cas9 protein derived from *S. aureus,* Cas9 protein derived from *Neisseria meningitides,* Cas9 protein derived from *Pasteurella multocida,* Cas9 protein derived from *Francisella novicida,* and the like, but it is not limited thereto. Information of Cas9 protein or gene of Cas9 protein can be obtained from a known database like GenBank of NCBI (National Center for Biotechnology Information).

Cas9 protein is an RNA-guided DNA endonuclease enzyme which induces breakage of a double stranded DNA. In order for Cas9 protein to cause DNA breakage after precise binding to a target nucleotide sequence, a short nucleotide sequence consisting of three nucleotides, which is known as PAM (Protospacer Adjacent Motif), should be present next to the target nucleotide sequence, and Cas9 protein causes the breakage by assuming the position between the 3^{rd} and the 4^{th} base pairs from the PAM sequence (NGG).

In the present invention, the guide RNA and endonuclease protein may function as RNA gene scissors (RNA-Guided Engineered Nuclease, RGEN) by forming a ribonucleic acid-protein (i.e., ribonucleoprotein) complex.

The CRISPR/Cas9 system employed in the present invention is a method of gene editing based on NHEJ (non-homologous end joining) mechanism in which insertion-deletion (InDel) mutation resulting from incomplete repair, which is induced during a process of DNA repair, is caused by introducing breakage of a double strand at specific site of a specific gene to be edited.

With regard to the method of producing a genome-edited rice plant according to the present invention, introducing guide RNA and endonuclease protein to a rice plant cell of the aforementioned step (a) may involve use of: a complex (i.e., ribinucleoprotein) of a guide RNA specific to the target nucleotide sequence in *OsBCAT2* gene derived from rice and an endonuclease protein; or a recombinant vector having a DNA encoding a guide RNA specific to the target nucleotide sequence in *OsBCAT2* gene derived from rice and a nucleic acid sequence encoding an endonuclease protein, but it is not limited thereto.

Furthermore, with regard to the method of producing a genome-edited rice plant according to one embodiment of the present invention, the target nucleotide sequence in *OsBCAT2* gene derived from rice may be the nucleotide sequence of SEQ ID NO: 3 or SEQ ID NO: 4, but it is not limited thereto.

The present invention further provides a genome-edited rice plant having enhanced tolerance to drought stress, which is produced by the aforementioned method of producing a genome-edited rice plant, and a genome-edited seed thereof.

The genome-edited rice plant according to the present invention is characterized in that, as the gene encoding BCAT2 protein derived from rice is knocked-out, the plant exhibits enhanced tolerance to drought stress.

The present invention still further provides a composition for controlling tolerance to drought stress in plant comprising, as an effective component, a gene encoding OsBCAT2 protein derived from rice.

The composition of the present invention comprises, as an effective component, a gene encoding OsBCAT2 protein derived from rice or a mutant of the gene encoding OsBCAT2 protein derived from rice, and, by transforming a plant cell with a recombinant vector containing a gene encoding OsBCAT2 protein derived from rice or a mutant of the gene encoding OsBCAT2 protein derived from rice, plant tolerance to drought stress can be controlled.

Hereinbelow, the present invention is explained in greater detail in view of the Examples. However, it is evident that the following Examples are given only for exemplification of the present invention and by no means the present invention is limited to the following Examples.

### Materials and Method

### 1. Plant materials

To generate a loss of function *OsBCAT2* (Os03g0231600, LOC_Os03g12890) mutants *(osbcat2),* rice (*Oryza sativa* L. ssp. Japonica cv. Dongjin) was edited using the CRISPR-Cas9 system through two guide RNAs. Guide RNA was designed via the web-based tool CRISPR RGEN Tools (http://www.rgenome.net). As described previous report, the method was performed (Chung et al. 2020, Int. J. Mol. Sci. 21(24);9606). We obtained *osbcat2* mutants with a total of 18 genotypes. Among these, we selected *osbcat2* mutants (#3, #4, #5, #10, and #15), which have premature stop codons before 89 amino acids were used in this research.

The Japonica rice cultivar Dongjin (*Oryza sativa* L.) grown under the same conditions as mutants *(osbcat2),* was used as the non-transgenic control.

**[Table 1]**

| sgRNAs for OsBCAT2 gene editing | |
|---|---|
| | Sequence (5'-3') |
| sgRNA1 | CGGGAGGAGCGCGCGCTTCG (SEQ ID NO: 3) |
| sgRNA2 | CGCGCTGGCCAGGGCCCTGC (SEQ ID NO: 4) |

### 2. Abiotic stress and phytohormone treatments

On a Murashige-Skoog (MS) solid medium, rice seeds *(Oryza sativa* cv. Dongjin) were germinated for four days at 28°C in the dark. The seedlings were then transported to a growth chamber with a 16 h light / 8 h dark cycle, 200 µmol m⁻² s⁻¹ of light intensity, and 70 % relative humidity. One-week-old seedlings were transferred to a Yoshida solution and grown for two more weeks for gene expression analysis. Non-transgenic (NT) (*Oryza sativa* L. ssp. Japonica cv. Dongjin) seedlings were used to study the phytohormone-dependent response of *OsBCAT2* and were transferred to 50 ml tubes containing 100 µM ABA (abscisic acid), JA (jasmonic acid), and SA (salicylic acid) solution (Sigma, USA). Seedlings were harvested every 2 hours until 6 hours after each hormone treatment for RNA extraction.

To confirm the transcription levels of the *OsBCAT2* gene under various abiotic stresses, non-transgenic (NT) plants were grown in soil for two weeks under standard greenhouse conditions (16 h light / 8 h dark cycles at 28~30°C). For stress treatments, remove the soil from the roots of the seedling completely, and drought stress was induced by air-drying the seedlings, while salinity stress was imposed by incubating the seedlings in water containing 400 mM NaCl at 28°C. Low-temperature stress was treated by incubating the seedlings in water at 4°C (±1.5°C).

### 3. Amino acid feeding and water deficit stress treatment

Non-transgenic plants (*Oryza sativa* cv. Dongjin) were sown on MS (Murashige and Skoog) media and incubated in a dark growth chamber for four days at 28°C. Seedlings were then transferred to a growth chamber with a light/dark cycle of 16 h light / 8 h dark at 30°C and grown to the two-week-old seedling. The seedlings were acclimatized in water for one day before being transferred to the Yoshida solution. They were then grown to three-week-old plants in Yoshida solution.

Three-week-old plants grown in Yoshida liquid media were fed with 10 mM randomly selected FAAs, including L-valine, leucine, and isoleucine (Sigma, USA) by dipping their roots into a corresponding FAA solution for 24 hrs. Plants pretreated with BCAA were harvested for amino acid analysis. Plants were then transferred to a 50 ml tube containing 30 mL of 21 % PEG 8000 (Sigma) solution. PEG-induced visual symptoms such as leaf rolling and wilting were monitored by imaging plants at the indicated time points using an α5000 (Sony) camera. Air-drying-induced drought phenotypes of FAA pretreated NT plants were recorded through relative water content (RWC) and symptoms analysis. Calculation formula of RWC (%) = (FW - DW)/(TW - DW) × 100. FW: fresh weight, DW: dry weight, TW: turgid weight.

### 4. RNA extraction and quantitative real-time PCR (qRT-PCR) analysis

To investigate the spatiotemporal gene transcript patterns of *OsBCAT2,* total RNA samples were extracted from the roots, stem, leaf, and flowers from different developmental stages of rice plants using a Hybrid-R RNA purification kit (GeneAll, Seoul, Korea) according to the manufacturer's instructions. To measure the expression levels of *OsBCAT2,* total RNA samples were isolated from the shoots and roots using a Hybrid-R RNA purification kit (GeneAll, Seoul, Korea). The cDNAs of total and/or immunoprecipitated RNAs were synthesized using the RevertAid^{™} First Strand cDNA Synthesis Kit with an oligo (dT) primer (Thermo Scientific, Waltham, MA, USA). 20 ng of cDNA was utilized as a template for qRT-PCR analysis. qRT-PCR was carried out using 2x qRT-PCR Pre-mix with 20x EvaGreen (SolGent, Seoul, Korea) and ROX dye (Promega, Madison, WI, USA). The amplification reactions were carried out at 95°C for 10 minutes, followed by 40 cycles at 95°C for 30 seconds, 60°C for 30 seconds, and 72°C for 30 seconds in a 20 µL volume mix containing 1 µL EvaGreen Mix. The rice *UBIQUITIN1* (Os06g0681400) were used as an internal standard.

**[Table 2]**

| Primer information used in the present invention | | | |
|---|---|---|---|
| Purpose | Gene ID | Gene Name | Sequence (5'-3') (SEQ ID NO:) |
| Clone | Os03g0231600 | *OsBCAT2* | F: |
| | | | TTGCTCCGTGGATCCATGGCTGCTGCT GCTGCTGC (5) |
| Clone | Os03g0231600 | *OsBCAT2* | R: |
| | | | AAAGCGGCCGCAAATATCTATTGCCA CTGTCCATCC (6) |
| RT-PCR | Os03g0231600 | *OsBCAT2* | F: GTGCACAAGACGTACCTGGA (7) |
| RT-PCR | Os03g0231600 | *OsBCAT2* | R: GGAGACGAGCCGTTCTTCAA (8) |
| RT-PCR | Os05g0586200 | *OsJarl* | F: GCTTCCACAACTCCACACCT (9) |
| RT-PCR | Os05g0586200 | *OsJarl* | R: CGCTTGATCGTTCCACGAAG (10) |
| RT-PCR | 0s05t0190500 | *OsVSP2* | F: GGGAACACTGCGACTCTACC (11) |
| RT-PCR | Os05t0190500 | *OsVSP2* | R: GCCCAACAGGCTGGAGTAAT (12) |
| RT-PCR | Os11t0605500 | *OsACX2* | F: CATGTGGTGGCCATGGGTAT (13) |
| RT-PCR | Os11t0605500 | *OsACX2* | R: ATCTCCAGCAACCTGCTGAA (14) |
| RT-PCR | Os02t0194700 | *OsLOX1* | F: CATGCCGTCCAAGATGCA (15) |
| RT-PCR | Os02t0194700 | *OsLOX1* | R: GGAGTGCGACGACAGGATGT (16) |
| RT-PCR | Os05t0304600 | *OsLOX7* | F: CCGATATCTATGCCCATTGGA (17) |
| RT-PCR | Os05t0304600 | *OsLOX7* | R: CCATCCCCCTCTTGATGAGA (18) |
| RT-PCR | Os08t0509100 | *OsLOX8* | F: GCAGCTCAGCGAGATGCA (19) |
| RT-PCR | Os08t0509100 | *OsLOX8* | R: CGTTGATCCGCATCGTGTAC (20) |
| RT-PCR | Os01g0165000 | *OsAPX2* | F: TCCTACGCCGACTTCTACCA (21) |
| RT-PCR | Os01g0165000 | *OsAPX2* | R: CGGCGTAATCCGCAAAGAAG (22) |
| RT-PCR | Os06g0115400 | *OsSOD1* | F: CAGGTTGAGGGAGTCGTCAC (23) |
| RT-PCR | Os06g0115400 | *OsSOD1* | R: GGTTGCCTCAGCTACACCTT (24) |
| RT-PCR | Os06g0143000 | *OsSOD2* | F: GTGAAGGCTGTTGTTGTGCT (25) |
| RT-PCR | Os06g0143000 | *OsSOD2* | R: GCCAGAGACACTTCCAGTCA (26) |
| RT-PCR | Os06g0727200 | *OsCAT* | F: TACTTCCCATCCCGCTACGA (27) |
| RT-PCR | Os06g0727200 | *OsCAT* | R: TCCTTACATGCTCGGCTTCG (28) |
| RT-PCR | Os06g0681400 | *OsUbi* | F: GCCAAGATCCAGGACAAGGA (29) |
| RT-PCR | Os06g0681400 | *OsUbi* | R: GCCATCCTCCAGCTGCTT (30) |

### 5. Drought stress treatment and stress measurement at the vegetative stage

For 4 days, *osbcat2* and NT (*Oryza sativa* L. ssp. Japonica cv. Dongjin) seedlings were germinated on the Murashige-Skoog (MS) medium in a dark growth chamber at 28°C. Before the transplant, germinated plants were acclimated to light conditions for one day. The 5-day-old seedlings were transplanted into soil pots (4×4×6 cm, three plants per pot) within a container (59×38.5×15 cm) and grown for five weeks in greenhouse conditions at the automatically controlled glass-greenhouse condition (light / dark cycle of 16 h light / 8 h dark at 30°C ± 3°C). Drought treatment for observation or measurement was uniformly imposed by withholding water and re-watering at the vegetative phase of rice. Soil moisture was monitored during the test and measured by a soil moisture sensor (SM159, Delta-T Devices Cambridge, United Kingdom).

To measure the drought stress of *osbcat2* and NT, we evaluated the efficiency of photosystems I and II through JIP test (Strasser, R.J., Tsimilli-Michael, M., Srivastava, A. (2004). Analysis of the Chlorophyll a Fluorescence Transient. In: Papageorgiou, G.C., Govindjee (eds) Chlorophyll a Fluorescence. Advances in Photosynthesis and Respiration, vol 19. Springer, Dordrecht. https://doi.org/10.1007/978-1-4020-3218-9_12). These data were recorded using a Handy-PEA fluorimeter (Plant Efficiency Analyzer Hansatech Instruments, UK) in dark conditions to impose optimal dark adaptation (at least 20 min). Ten leaves were collected and calculated using the Handy PEA software (version 1.31) and analyzed according to the calculation formula of the JIP test.

### 6. Analysis of agronomic traits of rice in a rice paddy field

To evaluate the agronomic characteristics and traits of *osbcat2* and NT rice plants, five independent T1 homozygous mutants and NT (Oryza sativa L. ssp. Japonica cv. Dongjin) plants were cultivated in the paddy field at the Kyungpook National University, Gunwi (128:34E/36:15N), Korea. We divided the paddy field into three sections to minimize the influence of microclimate, and each plot was planted with ten plants per line. The experimental environment was created for agronomic evaluation of *osbcat2* and NT plants under dehydration conditions. Plants were grown in an artificially created reservoir with a rain-off shelter. Water deficit conditions is imposed to plants before and after the heading phase by controlling water levels. When phenotypes caused by drought stress were observed in NT, re-irrigation was performed for recovery. The yield components of 10 plants per line from three different plots for the drought field conditions were measured.

### 7. DAB and NBT Staining

For DAB staining, 50 mg 3,3'-Diaminobenzidine (Sigma) was dissolved in 45 ml H₂O₂ then the pH was adjusted to 3.8 with 0.1 N HCl. To this solution, 25 µL of Tween20 (0.05 % v/v) and 2.5ml of Na₂HPO₄ (200 mM) were added and filled with H₂O₂ to make a total volume of 50 ml. Leaf sections of accurately 2.5 cm in length were cut and soaked in a 1 % DAB solution. After 30 min vacuum infiltrating, the immersed leaves were incubated in the dark for 20 h at room temperature. And then, the leaves were bleached by bathing and boiling ethanol until the brown spots appeared clearly. The area of brown spots represents the DAB reaction degree to H₂O₂.

In order to detect superoxide accumulation using a 0.2 % solution of NBT in a 10 mM potassium phosphate buffer (pH 7.7), roughly 2.5 cm-long leaf pieces were cut. After 15 minutes of vacuum infiltration, the submerged leaves were incubated at room temperature for overnight. After incubation, the leaves were fixed and clarified in alcoholic lacto-phenol (2:1:1, % ethanol: lactic acid:phenol) at 65°C for 30 minutes, rinsing with 50% ethanol, and then rinsing with water. In leaves, a blue precipitate form when NBT reacts with superoxide.

### Example 1. OsBCAT2 is a drought-responsive mitochondrial BCAA aminotransferase

For 12 genes including *BCATs* of *Arabidopsis thaliana* (*AtBCATs*) and *BCATs* of tomato (*SIBCATs*) that are taken as a subject, multiple amino acid sequence alignment was carried out. As a result of the phylogenetic analysis, very high homology was found among *BCAT* genes of *Arabidopsis thaliana,* tomato, and rice, including *AtBCAT2* and *SIBCAT2* that are known to be present in mitochondria. Amino acid sequence homology of BCAA aminotransferase domain among them was more than 80% and it was recognized that difference of the amino acids sequence was found only in the signal peptide sequence region at the N terminus which determined the subcellular localization of the protein.

In order to find the drought-induced branched-chain amino acid aminotransferase genes, we used RNA-sequencing (RNA-seq) in a previous study (Chung et al. 2016 BMC Genomics. 17:563), 5 drought-responsive (2 up and 3 down-regulated, respectively, with a log2 ratio ≥ 2.0 and ≤ -2.0) BCAA transferase-related gene were identified. Among these, *OsBCAT2* was up-regulated by drought stress.

To confirm the transcription levels in *OsBCAT2* in various abiotic conditions, 2-week-old rice seedlings were exposed to drought, high salt, abscisic acid (ABA), and low temperature. These analyses show that *OsBCAT2* transcript level was strongly induced within 6hr of exposure to drought stress in leaves than in other abiotic stress (Figure 1a). These results are similar to RNA-seq data. Moreover, specifically in roots, the transcript level significantly increased after phytohormone (ABA, JA, SA) treatment (Figure 1b). *OsBCAT2* might have multiple enzymatic roles in response to abiotic stress.

### Example 2. OsBCAT2 is involved in BCAA catabolism

Amino acids are involved in most metabolic pathways and known to regulate various developmental processes in plants. We performed the transcription analysis of *OsBCAT2* by qRT-PCR. The expression level was checked in various tissues at different developmental stages, starting from vegetative to developmental stage. These data showed that *OsBCAT2* transcripts were up-regulated from all the rice tissues. *OsBCAT2* showed higher expression during the developmental stage than at the vegetative stage, specifically in leaves in dark conditions (Figure 4). This transcript pattern is similar to that of the gene that is related to BCAA catabolism in Arabidopsis.

It is known that major enzymes involved in biosynthesis and degradation of BCAA are present in chloroplast and mitochondria, respectively. Accordingly, by expressing OsBCAT2-GFP fusion protein in rice protoplast, the inventors of the present invention investigated the subcellular localization of OsBCAT2 in order to speculate whether OsBCAT2 is involved in the synthesis or degradation of BCAA. As a result of confocal laser scanning microscopy analysis, the fluorescent signal of OsBCAT2-GFP protein was observed only from mitochondria. In addition, as a result of subcellular localization analysis, it was found that the expression of OsBCAT2 protein is similar to the expression of mitochondria marker (Figure 5). These results suggest that, in mitochondria, OsBCAT2 is involved in catabolizing BCAA.

### Example 3. Loss of OsBCAT2 confers enhanced tolerance to drought

A previous study about *OsDIAT* overexpressing transgenic plants showed that increasing BCAA content in leaf tissue confers enhanced tolerance to drought stress (US Patent US11,046,970B2). To investigate the biological functions of *osbcat2* mutants under drought stress, we designed two types of single guide RNA (sgRNA) for CRISPR-Cas9 systems technology to achieve knockout mutants in *OsBCAT2* gene and obtained homozygous five individual mutants (#3, #4, #5, #10, #15) (Figure 6a). Both sgRNAs were designed in the first exon, gRNA1 mutants (#4, #10) had 1-base, 2-base deletion and gRNA2 mutants (#3, #5, #15) had 1-base deletion in different DNA sequences, respectively. Sequencing analysis revealed mutation patterns in transgenic plants, including frameshift and premature stop codon (Figure 6b).

We conducted a drought tolerance test on *osbcat2* mutants to examine their response to drought stress and biological roles. In the greenhouse, 4-week-old plants were subjected to drought stress by withholding water for several days (Figure 7a). Soil moisture indicated a constantly decreasing for 3 days, indicating that the drought stress was uniformly applied to plants, 1 day after drought treatments, drought-induced damage was displayed in NT plants (Figure 7e). 2 to 3 days after exposure to drought conditions, most NT plants were induced unrecoverable symptoms from drought stress, including leaf wilting, rolling, and chlorosis. Whereas all *osbcat2* mutant lines exhibited resistance to the drought stress and far less drought-induced symptoms after 3 days of drought exposure, and they had a higher recovery rate than NT plant after re-watering. To further verify the drought tolerance of *osbcat2* mutants, we determined the Maximum quantum efficiency of PS II system (Fv/Fm) values and photosynthetic performance index (PI) of PS I and PS II, which represent photochemical efficiency under abiotic stress. The Fv/Fm value of NT plants significantly decreased 3 days after stress exposure. On the other hand, *osbcat2* mutants showed a slighter decrease in Fv/Fm value (Figure 7b). Also, the Performance Index value also dramatically decreased in NT plants, whereas the PI value of *osbcat2* was well controlled during drought exposure conditions (Figure 7c). These results indicated that *osbcat2* mutants had higher drought tolerance than NT plants.

### Example 4. BCAA levels are significantly accumulated in osbcat2 plants

It was reported that Arabidopsis plants increased BCAA in response to drought and osmotic stress. We evaluated whether BCAA levels in NT and *osbcat2* mutant are different under drought conditions. For High-performance liquid chromatography (HPLC) analysis, we exposed them (6-week-old plants) to drought stress conditions by air drying (6hr). We measured the branched-chain amino acid content under normal and drought conditions. Compared to normal conditions samples, BCAA levels significantly increased under drought stress conditions. Specifically, *osbcat2* mutants accumulated much higher BCAA than NT plants (Figure 8a). To further validate amino acid changes according to the drought treatment period, we exposed them to soil drought stress conditions for three days. Leucine content in *osbcat2* mutants was increased during first two days. Further, on three days, BCAA levels rapidly increased. On the other hand, BCAA content of NT plants was induced during first two days but decreased on the third day. Total BCAA content of *osbcat2* was substantially increased compared to NT plants under drought conditions (Figure 8b).

In order to further confirm whether the *OsBCAT2* gene is involved in BCAA catabolism, we analyzed BCAA content in dried rice seed. The result showed that BCAA content exponentially increased in *osbcat2* mutants than that in NT plants. Overall, these results indicate that drought stress leads to BCAA accumulation in rice plants and the expression of *OsBCAT2,* involving in BCAA catabolism, is induced by drought stress.

### Example 5. The external supply of branched-chain amino acids enhances osmotic shock tolerance in rice

To determine the effect of amino acid pre-treatment in osmotic stress tolerance, various amino acids (i.e., proline, alanine, aspartic acid, methionine, isoleucine, leucine, valine, and BCAA compounds) were applied to NT rice for 24 hours before osmotic stress treatment. The pre-treated plants were examined by HPLC analysis. The result showed that the internal amino acid level has been significantly elevated by external application (Figure 2a). Then, the pre-treated plants were applied with 21% PEG 8000 for simulating the osmotic stress. After the PEG treatment for 13 hours, symptoms of osmotic stress, including leaf curling and wilting, were observed from most NT plants pre-treated with amino acids, except BCAA and proline. Level of the symptoms was the strongest in Mock group followed by Ala, Asp, Met, Phe, BCAA, and Pro pre-treated group (Figure 2b). It was also shown that the exogenous BCAA pre-treatment can promote the regeneration of plants damaged by PEG treatment after re-watering and also alleviate the symptoms of osmotic shock.

Furthermore, to determine the effect of amino acid accumulation in rice under severe osmotic stress, an air-drying test was carried out. First, a pre-treatment with amino acids was carried out for 24 hours in the same manner as the treatment method described in the above. After the air-drying treatment for 10 hours, it appears that significant damages are caused by drought stress in all plants. However, the plant treated with BCAA or proline showed less damage compared to the plant groups which have been pre-treated with other amino acids (Figure 3a). The plant with mock treatment showed unrecoverable symptoms even after re-watering, while plant pre-treated with BCAA or proline showed recovery after re-watering. Based on these results, it was found that a pre-treatment with individual BCAA, a treatment with combination of BCAAs, and proline treatment can provide tolerance to drought stress, which has been caused by air drying. It was recognized that, similar to the PEG test described above, the BCAA and proline treatment groups can have recovery through a recovery process.

In order to further investigate the effect of supplying exogenous BCAA, level of moisture loss caused by air drying was monitored every hour. The results showed that the moisture loss was reduced by pre-treatment of any amino acid compared to that of plant with mock treatment (Figure 3b). In particular, the pre-treatment with 3 individual BCAAs, combination of those BCAAs, or proline showed much better property of maintaining the moisture content compared to plants treated with Ala, Asp, Met, and Phe (Figure 3c). In summary, BCAA accumulation in rice can provide the tolerance to osmotic stress.

### Example 6. Genome editing of OsBCAT2 improves agronomic traits under field drought conditions

The above study revealed that loss of function *osbcat2* mutant confers drought tolerance. To evaluate the yield performance of *osbcat2* mutants under drought stress conditions, *osbcat2* and NT plants were grown in the field conditions with rain-off shelters at Gunwi (128:34E/36:15N), Korea, for 100 days. Then, the sequential drought stress at the flowering and milk stage were applied by halting irrigation and drying 2-weeks. Under normal conditions, five independent *osbcat2* were slightly lower than NT in the number of spikelets (NS) and Filling rate (FR). On the other hand, under drought conditions, *osbcat2* significantly improved number of total seed (NTS) and total grain weight (TGW) (Figure 9a and Table 3) compared to NT.

**[Table 3]**

| **The Agronomic trait of *osbcat2* rice plants grown under normal and drought stress conditions Normal** | | | | | | | | |
|---|---|---|---|---|---|---|---|---|
| Constructs | Culm length (cm) | Panicle length (cm) | No. of panicles (/ hill) | No.of spikelets (/panicle) | No. of total spikelets (/ hill) | Filling rate (%) | Total grain weight (g) | 1000 grain weight (g) |
| **NT** | 81.45 | 21.38 | 12.10 | 99.57 | 1219.20 | 82.18 | 24.25 | 24.06 |
| *osbcat2* | 75.03 | 20.49 | 12.56 | 86.08 | 1310.25 | 69.15 | 20.99 | 21.82 |
| %Δ | -7.89 | -4.14 | 3.80 | -13.55 | 7.47 | -15.86 | -13.43 | -9.31 |
| *p val* | 0.042 | 0.098 | 0.284 | 0.004 | 0.216 | 0.002 | 0.089 | 0.076 |

| **Drought** | | | | | | | | |
|---|---|---|---|---|---|---|---|---|
| Constructs | Culm length (cm) | Panicle length (cm) | No. of panicles (/ hill) | No.of spikelets (/panicle) | No. of total spikelets (/ hill) | Filling rate (%) | Total grain weight (g) | 1000 grain weight (g) |
| **NT** | 62.54 | 14.25 | 9.26 | 72.17 | 862.49 | 51.74 | 11.06 | 18.99 |
| ***osbcat2*** | 70.16 | 15.57 | 11.14 | 72.81 | 1043.32 | 49.56 | 12.89 | 19.58 |
| %Δ | 12.18 | 9.26 | 20.30 | 0.89 | 20.97 | -4.21 | 16.55 | 3.11 |
| *p* val | 0.056 | 0.129 | 0.044 | 0.370 | 0.086 | 0.084 | 0.023 | 0.519 |

### Example 7. JA induced antioxidant gene meditates drought tolerance of osbcat2 mutants

Proline is one of the best characterized amino acids involving in drought tolerance in plants, and the mechanism of abiotic stress responses by proline has been studied well. However, the tolerance mechanism against abiotic stress by BCAA has been poorly understood so far.

It has been well documented that biological active form of JA is JA-Ile, conjugated form with isoleucine in plants, and it is mediated by *JASMONATE RESISTANT1* (*JAR1*). To identify the function of the *OsBCAT2* in JA signaling, we checked the transcription level of rice *JAR1* under drought stress. *JAR1* expression level was significantly higher in *osbcat2* than in NT plants (Figure 10a). Also, we confirm that expression level of JA-responsive genes enhanced in *osbcat2* compared to NT plants (Figure 10b and c), suggesting that *OsBCAT2* mutation promotes JA signaling.

Expression of JA responsive antioxidant genes, such as APX, SOD, and POD, was highly up-regulated in *osbcat2* (Figures 11d). Then we conducted hydrogen peroxide (H₂O₂) and superoxide anion (O₂) analysis in *osbcat2* mutants to check ROS (Reactive Oxygen Species) accumulation. DAB and NBT staining, which provokes irreparable brown and blue products with reacting H₂O₂ and O₂- respectively, showed that *osbcat2* mutants had less accumulated H₂O₂ than NT in response to drought stress (Figure 11). Summarizing these results, drought stress-induced ROS was well controlled by JA-induced antioxidant genes in *osbcat2,* which confers drought tolerance.

SEQUENCE LISTING

SEQ ID N°1
Oryza sativa BCAT2 nucleotides
ATGGCTGCTGCTGCTGCTGCGTCGTCCGCGAAGCGCGCGCTCCTCCCGTGGGCA
CGCGACGCCCACCACGCGCTGGCCAGGGCCCTGCAGGGATGCGGCGGCGGCGG
CCTCGGTCTCCGCGGGGCGCTCCCGACGGCCGGAGGCAGGTGGTCTCTGCTCCAGTG
CCGGTGGAGGTCGTCGCTGCCGCAGCTCGACTCCGCCGACAGGTCCGATGAGGAAA
GCGGCGGCGAAATCGACTGGGACAACCTGGGGTTCGGGCTGACGCCGACCGACTAC
ATGTACGTCATGCGGTGCTCGCTGGAGGACGGCGTCTTCTCCCGCGGCGAGCTCAGC
CGCTACGGCAACATCGAGCTCAGCCCCTCCTCCGGCGTCATCAACTACGGCCAGGG
GCTCTTCGAGGGTCTGAAGGCGTACAGGGCGGCGAACCAACAGGGGTCGTACATGC
TGTTCCGGCCGGAGGAGAACGCGCGGCGGATGCAGCACGGCGCCGAGCGCATGTGC
ATGCCGTCGCCGTCGGTGGAGCAGTTCGTCCACGCCGTCAAGCAGACCGTCCTCGCC
AACCGCCGCTGGGTGCCACCGCAAGGAAAGGGGGCGCTGTACATCAGGCCGCTGCT
CATCGGGAGCGGACCGATTCTCGGGCTGGCTCCCGCCCCGGAGTACACGTTCCTCAT
CTACGCCGCACCGGTTGGAACGTACTTCAAGGAGGGTCTAGCGCCGATAAACCTTGT
CGTAGAGGACTCGATACACCGGGCCATGCCGGGCGGCACCGGCGGGGTCAAGACGA
TCACCAACTACGCGCCGGTGCTCAAGGCGCAGATGGACGCCAAGAGCAGAGGGTTC
ACTGACGTGCTGTACCTCGACGCGGTGCACAAGACGTACCTGGAGGAGGCCTCCTC
CTGCAACCTCTTCATCGTCAAGGACGGCGTCGTCGCCACGCCGGCCACCGTGGGAAC
CATCCTGCCGGGGATCACGCGCAAGAGCGTCATCGAGCTCGCCAGGGACCGCGGCT
ATCAGCAGGTTGAAGAACGGCTCGTCTCCATCGACGATCTGGTCGGCGCAGACGAG
GTGTTCTGCACCGGAACAGCGGTGGTCGTTGCCCCAGTATCGAGTGTTACTTACCAT
GGGCAAAGGTACGAGTTCAGGACTGGACATGACACGTTATCGCAGACACTGCACAC
GACTCTGACGTCCATCCAGATGGGCCTGGCTGAGGACAAGAAAGGATGGACAGTGG
CAATAGATTAA

SEQ ID N°2
Oryza sativa BCAT2 amino acids

MAAAAAAASSAKRALLPWARDAHHALARALQGCGGGGGLGLRGALPTAGGRWSLLQ
CRWRSSLPQLDSADRSDEESGGEIDWDNLGFGLTPTDYMYVMRCSLEDGVFSRGELSR
YGNIELSPSSGVINYGQGLFEGLKAYRAANQQGSYMLFRPEENARRMQHGAERMCMPS
PSVEQFVHAVKQTVLANRRWVPPQGKGALYIRPLLIGSGPILGLAPAPEYTFLIYAAPVG
TYFKEGLAPINLVVEDSIHRAMPGGTGGVKTITNYAPVLKAQMDAKSRGFTDVLYLDA
VHKTYLEEASSCNLFIVKDGVVATPATVGTILPGITRKSVIELARDRGYQQVEERLVSID
DLVGADEVFCTGTAVVVAPVSSVTYHGQRYEFRTGHDTLSQTLHTTLTSIQMGLAEDK
KGWTVAID

SEQ ID N°31
Figure 6 – OriBCAT gRNA1 WT
CCGCGTTGCGCGCTCCTCCCG

SEQ ID N°32
Figure 6 – OriBCAT gRNA1 #4
CCGCGAGCGCGCTCCTCCCG

SEQ ID N°33
Figure 6 – OriBCAT gRNA1 #10
CCGCGAAGCGCGCTCCTCCCG

SEQ ID N°34
Figure 6 – OriBCAT gRNA2 #WT
CGCGCTGGCCAGGGCCCTGCAGG

SEQ ID N°35
Figure 6 – OriBCAT gRNA2 #3
CGCGCTGGCCAGGGCCTGCAGG

SEQ ID N°36
Figure 6 – OriBCAT gRNA2 #5

## Claims

1. A method of controlling tolerance to drought stress in a plant comprising regulating expression of a gene encoding OsBCAT2 (BRANCHED-CHAIN AMINO ACID AMINOTRANSFERASE 2) protein derived from rice in a plant cell.

2. The method of controlling tolerance to drought stress according to Claim 1, wherein the BCAT2 protein derived from rice consists of the amino acid sequence of SEQ ID NO: 2.

3. The method of controlling tolerance to drought stress according to Claim 1, wherein enhanced tolerance to drought stress compared to a non-transgenic plant is obtained by inhibiting expression of a gene encoding OsBCAT2 protein derived from rice in a plant cell by transformation of the plant cell with a recombinant vector containing the gene encoding OsBCAT2 protein derived from rice.

4. The method of controlling tolerance to drought stress according to Claim 1, wherein enhanced tolerance to drought stress is obtained by knocking-out the gene encoding OsBCAT2 protein derived from rice by using gene editing system.

5. A method of producing a transgenic plant having controlled tolerance to drought stress comprising:
transforming a plant cell with a recombinant vector containing a gene encoding OsBCAT2 protein derived from rice; and
regenerating a transgenic plant from the transformed plant cell.

6. The method of producing a transgenic plant according to Claim 5, wherein enhanced tolerance to drought stress compared to a non-transgenic plant is obtained by inhibiting expression of the gene encoding OsBCAT2 protein derived from rice.

7. A transgenic plant having controlled tolerance to drought stress produced by the method of Claim 5 or 6.

8. A transgenic seed of the plant according to Claim 7.

9. A method of producing a genome-edited rice plant having enhanced tolerance to drought stress comprising:
(a) carrying out genome editing by introducing guide RNA specific to target nucleotide sequence in a gene encoding OsBCAT2 protein derived from rice, and endonuclease protein to a rice plant cell; and
(b) regenerating a plant from the genome-edited rice plant cell.

10. The method of producing a genome-edited rice plant according to Claim 9, wherein the introduction of guide RNA and endonuclease protein to a rice plant cell of the step (a) involves use of: a recombinant vector containing a DNA which encodes guide RNA specific to target nucleotide sequence in *OsBCAT2* gene derived from rice and a nucleic acid sequence which encodes an endonuclease protein: or a complex (i.e., ribonucleoprotein) between guide RNA specific to target nucleotide sequence in *OsBCAT2* gene derived from rice and an endonuclease protein.

11. The method of producing a genome-edited rice plant according to Claim 9, wherein the target nucleotide sequence in *OsBCAT2* gene derived from rice is the nucleotide sequence of SEQ ID NO: 3 or SEQ ID NO: 4.

12. A genome-edited rice plant having enhanced tolerance to drought stress produced by the production method of any one of Claims 9 to 11.

13. A genome-edited seed of the rice plant according to Claim 12.

14. A composition for controlling tolerance to drought stress in a plant comprising, as an effective component, a gene encoding OsBCAT2 protein derived from rice.
